# EUROPEAN PATENT APPLICATION

(11) **EP 4 057 005 A1**
(43) Date of publication of application: **14.09.2022**
(21) Application number: 21162126.3
(22) Date of filing: 11.03.2021
(51) Int. Cl.: G01N 33/574

(54) **BIOMARKER FOR USE IN CANCER THERAPY**

(71) Applicant: Martin-Luther-Universität Halle-Wittenberg, 06108 Halle (Saale) (DE)
(72) Inventor: SELIGER, Barbara, 06114 Halle (Saale) (DE); VAXEVANIS, Christoforos, 06108 Halle (Saale) (DE); SUBBARAYAN, Karthikeyan, 06122 Halle (Saale) (DE); WICKENHAUSER, Claudia, 04668 Grimma (DE); BAUER, Marcus, 04103 Leipzig (DE)
(74) Representative: Vossius & Partner Patentanwälte Rechtsanwälte mbB

(57) **Abstract**

The present invention is in the field of medicine, particularly in the field of companion diagnostics and personalised medicine. It relates to means, methods, kits, systems and uses thereof for identifying the probability of a subject suffering from cancer to benefit from a specific personalized cancer therapy, particularly a cancer immunotherapy, based on the determination of the level of a small leucine-rich proteoglycan (SLRP), preferably biglycan (BGN), or a fragment thereof in a sample of said subject. Equally, the present invention relates to means, methods, kits and systems uses thereof for application of a customized personalized therapy option in a subject based on the identification of a targetable proteoglycan (SLRP), preferably biglycan (BGN), or a fragment thereof in a sample of said subject. Particularly, the cancer may be a hematopoietic cancer.

SEQUENCE LISTING

<110> MARTIN-LUTHER-UNIVERSITÄT HALLE-WITTENBERG

<120> BIOMARKER FOR USE IN CANCER THERAPY

<130> AD3830 EP BLN

<160> 2

<170> BiSSAP 1.3.6

<210> 1
<211> 368
<212> PRT
<213> Artificial Sequence

<220>
<223> BGN Homo sapiens

<400> 1

<210> 2
<211> 238
<212> PRT
<213> Artificial Sequence

<220>
<223> isotype BGN Homo sapiens

<400> 2
Met Trp Pro Leu Trp Arg Leu Val Ser Leu Leu Ala Leu Ser Gln Ala
1               5                   10                  15
Leu Pro Phe Glu Gln Arg Gly Phe Trp Asp Phe Thr Leu Asp Asp Gly
            20                  25                  30
Pro Phe Met Met Asn Asp Glu Glu Ala Ser Gly Ala Asp Thr Ser Gly
        35                  40                  45
Val Leu Asp Pro Asp Ser Val Thr Pro Thr Tyr Ser Ala Met Cys Pro
    50                  55                  60
Phe Gly Cys His Cys His Leu Arg Val Val Gln Cys Ser Asp Leu Gly
65                  70                  75                  80
Leu Lys Ser Val Pro Lys Glu Ile Ser Pro Asp Thr Thr Leu Leu Asp
                85                  90                  95
Leu Gln Asn Asn Asp Ile Ser Glu Leu Arg Lys Asp Asp Phe Lys Gly
            100                 105                 110
Leu Gln His Leu Tyr Val Arg Ser Trp Glu Glu Pro Ala Gly Leu Gln
        115                 120                 125
Gln Arg Ala Gly Val Arg Ala Leu Val Leu Val Asn Asn Lys Ile Ser
    130                 135                 140
Lys Ile His Glu Lys Ala Phe Ser Pro Leu Arg Lys Leu Gln Lys Leu
145                 150                 155                 160
Tyr Ile Ser Lys Asn His Leu Val Glu Ile Pro Pro Asn Leu Pro Ser
                165                 170                 175
Ser Leu Val Glu Leu Arg Ile His Asp Asn Arg Ile Arg Lys Val Pro
            180                 185                 190
Lys Gly Val Phe Ser Gly Leu Arg Asn Met Asn Cys Ile Glu Met Gly
        195                 200                 205
Gly Asn Pro Leu Glu Asn Ser Gly Phe Glu Pro Gly Ala Phe Asp Gly
    210                 215                 220
Leu Lys Leu Asn Tyr Leu Arg Ile Ser Glu Ala Lys Leu Thr
225                 230                 235

## Description

### FIELD OF THE INVENTION

The present invention is in the field of medicine, particularly in the field of companion diagnostics and personalised medicine. It relates to means, methods, kits, systems and uses thereof for identifying the probability of a subject suffering from cancer to benefit from a specific personalized cancer therapy, particularly a cancer immunotherapy, based on the determination of the level of a small leucine-rich proteoglycan (SLRP), preferably biglycan (BGN), or a fragment thereof in a sample of said subject. Equally, the present invention relates to means, methods, kits and systems uses thereof for application of a customized personalized therapy option in a subject based on the identification of a targetable proteoglycan (SLRP), preferably biglycan (BGN), or a fragment thereof in a sample of said subject. Particularly, the cancer may be a hematopoietic cancer.

### BACKGROUND OF THE INVENTION

Hematopoietic cancers are neoplastic diseases commonly associated with chromosomal abnormalities that affect the blood, bone marrow, lymphatic system, spleen and thymus (Taylor et al. Precision Hematology, 130(4):410-423; 2017). They are divided in lymphatic and hematopoietic neoplasms as well as in diseases involving the blood (leukemia) or the lymph nodes (lymphomas), and more accurately by the WHO Classification (published in 2001 and updated in 2008 and 2016). The often poor prognosis of patients suffering from hematopoietic cancer and the heterogeneous outcome after treatment in said patients show that there is an urgent need for guidance in making clinical decisions on the treatment of said patients.

Accordingly, myelodysplastic syndromes (MDS) refer to a group of clonal hematopoietic cancers, characterized by ineffective hematopoiesis and peripheral blood cytopenias (Corey S 2007, PMID: 17251918) that can be indolent or rapidly progressive with complications secondary to profound cytopenia and the risk of evolution into acute myeloid leukemia (AML). Acute myeloid / myelogenous leukemia (AML) (Ghobrial IM 2018, PMID: 29311715; Steensma DP 2015, PMID: 25931582) is a heterogeneous hematological malignancy involving the clonal expansion of myeloid blasts in the bone marrow and peripheral blood. According to the World Health Organization (WHO) an estimated 18.860 people were diagnosed in the USA in 2014, 10.460 of whom died from their disease. The median age at diagnosis was 66 years, with 54% patients over 65 years, and 33% over 75 years. Of those diagnosed at a later age, the diagnosis was often associated with underlying myelodysplastic syndromes (MDS), sometimes linked to cancer chemotherapy and radiotherapy exposure (Union for International Cancer Control; 2014 Review of Cancer Medicines on the WHO List of Essential Medicines).

Diagnosis of MDS relies mainly on morphologic peripheral blood and bone marrow findings. However, multifactorial pathogenic features with diverse cytogenetic, molecular and epigenetic alterations are associated with a variable clinical presentation and outcome (Greenberg PI 2012, PMID: 22212119_Molecular and genetic). Chronic inflammatory diseases associated with activated immune signaling pathways often precede the clinical manifestation of MDS suggesting an aetiopathogenetic link between chronic immune signaling, impaired stem cell quality and alteration of the stem cell microenvironment (Esplin BL 2011, PMID: 21441445). There is growing evidence of immune deregulation during the course of this disease (Winter S 2020, PMID: 32058844; Komrokji RS 2016, PMID: 26875020), such as overexpression of immune-related genes in hematopoietic stem and progenitor cells (HSPC) (Hofmann WK 2002, PMID: 12411319; Glenthoj A 2016, PMID: 27314337). However, the interrelationship between chronic immunologic stimulation and initiation as well as progression of MDS to secondary acute myeloid leukemia (sAML) remains largely unknown. Thus, a connection between immune deregulation (Pellagatti A 2010, PMID: 20220779; Fozza C 2018, PMID: 28449370) and established prognostic parameters, such as bone marrow (BM) blast count, cytogenetic alterations and the degree of peripheral cytopenia, which are all integrated in the Revised International Prognostic Score System (IPSS-R) (Greenberg PI 2012, PMID: 22740453; Kristinsson SY 2011, PMID: 21690473), might contribute to a better understanding of the pathogenesis and the consequences of altered immunologic features in this disease.

The International Prognostic Scoring System (IPSS) is the most commonly referenced risk stratification schema and was developed to assess risk at the time of diagnosis. The IPSS incorporates specific cytogenetic abnormalities, the percentage of marrow blasts, and the number of hematopoietic lineages involved in the cytopenia. A 5-category revised IPSS (IPSS-R) was developed which further subdivides cytogenetic abnormalities and increased the weight of higher blast percentages. In this context, low risk MDS are related to higher levels of CD8⁺ cytotoxic T lymphocytes (CTL) and lower levels of FoxP3⁺ regulatory T cells (Treg), while the frequency of CTL and Treg is inversely correlated in high risk MDS (Schanz T 2012, PMID: 22331955; Wang C 2018, PMID: 29458780; Mailloux AW 2012, PMID: 22875800). Recently, a leukemia subtype specific immunologic landscape was described suggesting disease-specific immune regulation (Brück et al. Myeloid Neoplasia, Blood Adv 4(2):274-286; 2020).

Two systems have been used to classify AML - the earlier French-American-British (FAB) classification and the more recent World Health Organization (WHO) classification. In the 1970-80s, French, American, and British leukemia experts divided AML into 8 subtypes (M0 to M7), based on the type of cell from which the leukemia developed and how mature the cells were. These subtypes are M0 referring to undifferentiated AML, M1 referring to AML without maturation (poorly differentiated), M2 referring to AML with maturation (more differentiated), M3 (acute promyelocytic leukemia (APML), M4 referring to acute myelomonocytic leukemia (AMML), M5 referring to acute monocytic leukemia, M6 referring to acute erythroblastic leukemia and M7 referring to acute megakaryoblastic leukemia. The WHO classification was revised in 2008 and classifies AML as described in detail herein below.

The majority of patients suffering from AML have a poor prognosis, indicating that it is still challenge to find an optimal therapy for a patient. There is no consensus regarding the optimal therapy for patients, particularly older patients. According to the WHO, older patients (over 60) do not fare well as younger patients as they are more likely to have unfavorable chromosome abnormalities as well as having comorbid medical conditions that can make it harder to use intense chemotherapy regimens. Older patients also suffer more from AML secondary (sAML) to previous myelodysplastic syndrome (MDS) which confers a worse prognosis.

Immunotherapy has been used forthe treatment of both solid and hematologic malignancies, however, the application of checkpoint inhibition immunotherapies for the treatment of MDS and AML has not been as efficient as expected while chimeric antigen receptor (CAR) T - cell therapy and natural killer cell based immunotherapy are currently approved in several studies (PMID: 33287858; PMID: 33384686; PMID: 31779970; PMID: 30926092).

The primary barrier currently preventing an efficient immunotherapy in patients suffering from MDS and AML is the lack of efficient and tumorspecific, potentially targetable biomarkers indicating a patient's benefit to receive a customized immunotherapy. The main reason for this lack in efficiency is that currently known targets on malignant blasts are shared with healthy hematopoietic stem cells or their progenitors. Further, a prerequisite to implement successfully an immunotherapy is a biomarker or target antigen, for which a depletion can be clinically tolerated. In detail, an enhanced targeting of a biomarker or target antigen present on cancer cells and non-neoplastic hematopoietic progenitors would lead to a life - threatening prolonged myeloid depletion. Therefore, solutions have to be urgently developed in order to circumvent these limitations by creating novel biomarkers or targets specifically expressed on cancer blasts, but not on normal hematopoietic stem cells (Cummins and Gill; Semin Hematol. 56(2):155-163; 2019)).

The inventors investigated proteoglycans which are normally embedded in the extracellular matrix (ECM). Small leucin rich proteoglycans (SLRP) are ubiquitous ECM components, which are involved in the structural organization of matrix. In the context of cancer initiation and progression they are also strongly involved in the regulation of cancer cell proliferation, angiogenesis and migration. The inventors surprisingly identified that a small leucin rich proteoglycan (SLRP) - an ubiquitous ECM component involved in the structural organization of matrix - biglycan (BGN) is highly expressed in AML blasts, but not in normal hematopoietic progenitors, therefore indicating that biglycan might be a targetable biomarker in this disease.

The technical problems underlying the present invention are the identification of a customized therapy option for patients suffering from hematopoietic cancer, particularly selected from MDS and AML, and thereby guiding clinical decisions in the treatment of said cancer.

The technical problems are solved by the underlying the present invention, particularly by (i) the accurate identification of a proteoglycan, (ii) the provision of a suitable target structure and (iii) the evaluation of the target structure for guiding clinical decisions in the treatment of MDS and AML.

The solution to these problems are provided by the embodiments as defined herein below and as characterized in the claims.

### BRIEF DESCRIPTION OF THE INVENTION

The inventors identified a new biomarker capable to efficiently discriminate AML / MDS cancer blasts from normal hematopoietic stem / precursor cells. This finding is the prerequisite for the design of a customized therapy option for patients suffering from hematopoietic cancer, particularly selected from MDS and AML, and includes the development of targetable effectors of glycoimmune checkpoint inhibitors to receive a specific therapy, particularly a cancer immunotherapy. This said biomarker is selected from a small leucine-rich proteoglycan (SLRP), preferably biglycan (BGN), or a fragment thereof.

The data presented herein demonstrate that a small leucine-rich proteoglycan (SLRP), preferably biglycan (BGN), or a fragment thereof provides an efficient biomarker for identifying targetable MDS/AML cancer blasts and induce a customized checkpoint immune therapy.

The present application relates to a method for identifying the cellular expression level of a small leucine-rich proteoglycan (SLRP), preferably biglycan (BGN), or a fragment thereof in cancer cells, preferably cancer blasts of AML and MDS, wherein said level of SLRP, preferably BGN, in the cancer blasts of the subject to be identified is indicative of a need of said subject to receive a cancer therapy, particularly a biomarker specific cancer immunotherapy.

Equally, the present application relates to a method for development of a substance with the potential of specific inhibition of small leucine-rich proteoglycan (SLRP), preferably biglycan (BGN), or a fragment thereof to receive a cancer therapy, particularly a biomarker specific cancer immunotherapy.

In this context, the expression level of a small leucine-rich proteoglycan (SLRP), preferably biglycan (BGN), or a fragment thereof may be determined in a great many of healthy bone marrow probes and AML / MDS bone marrows and compared to each other, as further outlined herein below. Hence, the invention further pertains to a method for identifying those patients benefitting from a individualized precision therapy, particularly a cancer immunotherapy, wherein an increased expression level of SLRP, preferably BGN, or a fragment thereof in the tumor blasts from the subject to be identified compared to a reference level is indicative of the therapeutic success by targeting of said subjects.

In particular, the present invention relates to the following:
A method for identifying the need of a subject suffering from or being at risk of developing cancer to receive a cancer therapy, particularly a cancer immunotherapy, comprising determining the level of a small leucine-rich proteoglycan (SLRP), preferably biglycan (BGN), or a fragment thereof in a sample of the subject to be identified, wherein said level of SLRP, preferably BGN, in the sample of the subject to be identified is indicative of a need of said subject to receive a cancer therapy, particularly a cancer immunotherapy.

Equally, the present invention relates to a method for the diagnosis, prognosis, risk assessment, risk stratification, therapy control and/or post-operative control of cancer in a subject comprising determining the level of a small leucine-rich proteoglycan (SLRP), preferably biglycan (BGN), or a fragment thereof in a sample of the subject to be identified, wherein said level of SLRP, preferably BGN, or a fragment thereof is indicative of said subject to suffer from or being at risk of developing cancer and/or a need of said subject to receive a cancer therapy, particularly a cancer immunotherapy.

Preferably, the method further comprises comparing the determined level of a SLRP, particularly BGN, or a fragment thereof to a reference level, particularly the reference level comprises the level of SLRP, particularly BGN, or a fragment thereof in a sample of at least one healthy subject, particularly the mean level of the SLRP, preferably BGN, or a fragment thereof in samples obtained from a healthy population.

More preferably, an increased level of SLRP, preferably BGN, or a fragment thereof in the sample from the subject to be identified compared to the reference level is indicative of a need of said subject to receive a cancer therapy, particularly a cancer immunotherapy. Preferably, SLRP, particularly BGN, or a fragment thereof is expressed on the cell surface, particularly on the cell surface of hematopoietic cells.

Preferably, SLRP is selected from the group consisting of BGN and decorin (DCN), more particularly SLRP is BGN.

Preferably, the level of SLRP, particularly BGN, or a fragment thereof in the sample from the subject to be identified of more than 2 fold compared to the reference level is indicative of a need of said subject to receive a cancer therapy, particularly a cancer immunotherapy. Preferably, the sample is selected from the group consisting of a blood sample, particularly a peripheral blood sample and serum, and a sample from bone marrow.

Preferably, the cancer is a hematopoietic cancer selected from one or more of myelodysplastic syndrome (MDS), acute myeloid leukemia (AML), particularly secondary acute myeloid leukemia (sAML), acute lymphoid leukemia (ALL), chronic lymphocytic leukemia (CLL), acute leukemia, B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, non-Hodgkin's lymphoma, Hodgkin's lymphoma, plasmablastic lymphoma, plasmacytoid dendritic cell neoplasm, and Waldenstrom macroglobulinemia. More preferably, the cancer is selected from the group consisting of myelodysplastic syndrome (MDS) and acute myeloid leukemia (AML), particularly secondary acute myeloid leukemia (sAML) chronic myeloid leukemia (CML), acute lymphocytic leukemia (ALL) and chronic lymphocytic leukemia (CLL).

Even more preferably, the cancer is MDS or AML, particularly sAML.

Equally, the present invention relates to the se of a small leucine-rich proteoglycan (SLRP), preferably biglycan (BGN), or a fragment thereof for identifying the need of a subject suffering from or being at risk of developing cancer to receive a cancer therapy, particularly a cancer immunotherapy, according to the methods of the present invention.

### DESCRIPTION OF FIGURES

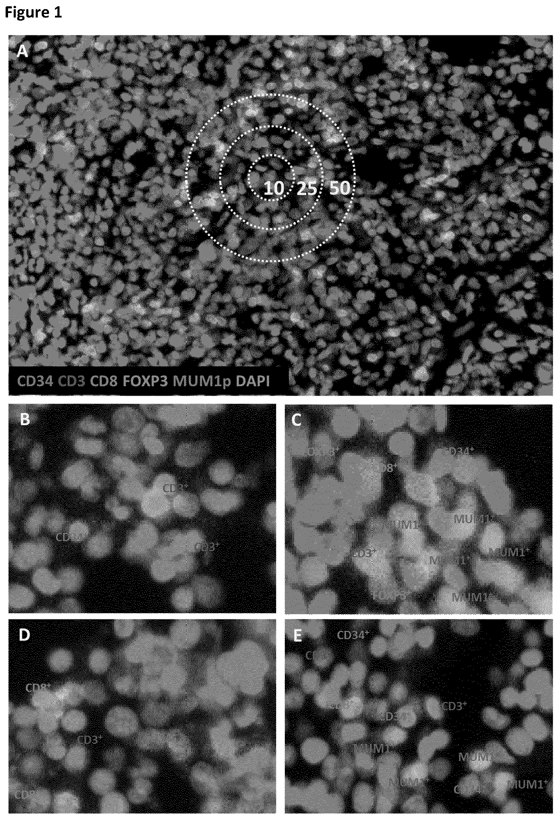

**Figure 1****: Multicolor analysis of BM biopsies performing MSI.** All Bone marrow biopsies (BMB) samples were stained with a five-color antibody panel consisting of CD34 (red), CD3 (green), CD8 (yellow), FOXP3 (turquoise), MUMlp (orange) antibodies and counterstained with DAPI (blue). (A) MDS with multilineage dysplasia without excess of blasts. Intercellular distance algorithm with four applied categories: direct intercellular contact (i), cells within a radius of 10 µm (ii), 25 µm (iii) and 50 µm (iv). (B) and (D) show control samples with few CD3⁺CD8⁻ T cells, but no other T cell and B/plasma cell subpopulations in close proximity of the CD34⁺ HSPC. However, in the BMB samples of MDS patients shown in the figures (C) and (E) many different T and B/plasma cell subpopulations were detected in close proximity of the CD34⁺ blasts.
**Figure 2****:** MDS-L cells were stained with an 8 color panel (CD34 - Texas Red, CD33 - PE, lin - FITC, CD38 - PerCP Cy5.5, CD45RA - APC, CD123 - APC-Fire, IL1RAcP - PE vio 770, Zombie aqua) and further analysed in subpopulations following the gating strategy depicted in the figure. Subpopulations were sorted using a FACS Aria Fusion machine and their BGN protein levels were identified through Western blot. SC1 and SC2 subpopulations with a phenotype similar to *in vivo* MDS stem cells (PMID: 30510255) appear to be the major BGN expressing cells.
**Figure 3****: BGN surface staining of 4 AML and 1 MDS (MDS-L) cell line.** Positivity was calculated based on the fluorescence levels of the isotype control used. BGN is detected on the surface of the cells.
**Figure 4****: BGN surface staining in the PBMCs acquired from the blood of two healthy donors (A7, CV).** Positivity was calculated based on fluorescence levels of the isotype control used.

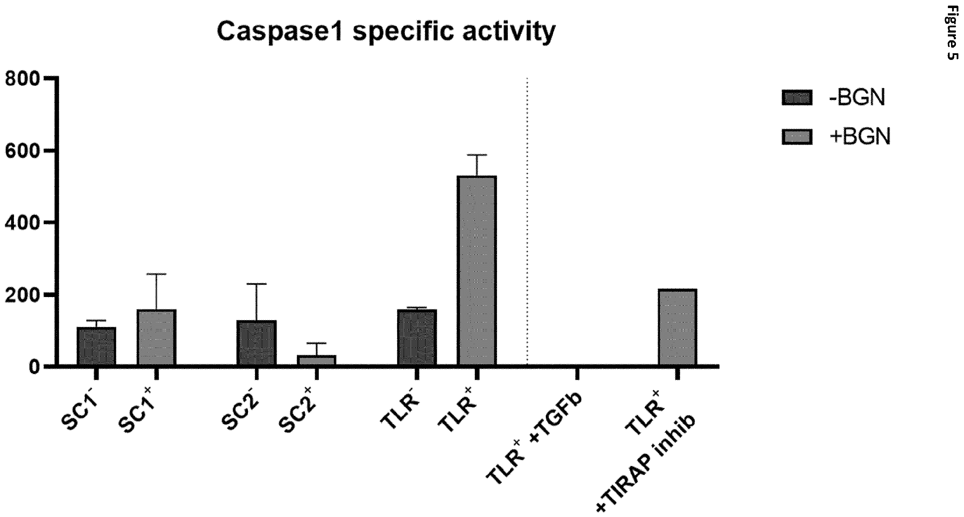

**Figure 5****: BGN-TLR4 mediated activation of the inflammasome on the MDS-L cell line as detected by caspase 1 activity.** MDS-L subpopulations were sorted and directly incubated overnight with 1µg/mL soluble BGN. Caspase 1 activity was measured forthe 3 subpopulations of interest (SC1, SC2, TLR4) and was found statistically higher only for the TLR4 subpopulation after biglycan stimulation. Additionaly, the TLR4 subpopulation was cultured in the presence of possible inhibitors of this pathway (TGF-b1, TIRAP inhibitor, IFN-y, IFN-a) with TGFb1 and IFN-y showing the strongest inhibition. Moreover, TLR4 cells were also cultured with the addition of supernatant from the BGN expressing SC2 subpopulation, or directly cocultured with SC2 cells resulting in high caspase 1 activity without the addition of recombinant human BGN.
**Figure 6****:** Correlation of BGN h score (taking into account BGN positive cells and staining intensity) and age within the bone marrow of MDS/sAML patients and healthy donors as determined by immunohistochemistry.
**Figure 7****:** Immunohistochemistry staining of BGN the bone marrow from two MDS patients demonstrating the range of intensity. On the left, the biopsy of an MDS patient within healthy BGN expression range can be observed, in comparison to the highly expressing patient on the right.
**Figure 8****:** Dot plots and ROC curves demonstrating the diagnostic value of BGN expression for MDS/sAML, taking into account only percentage of BGN positive cells (BGN%), or the BGN h score (cell positivity and intensity of staining). For both BGN% and BGN hscore the cutoff with maximum sensitivity and specificity is at 12,5 (Sens=0,69 Spec=1), with maximum values for healthy donors at hscoreₘₐₓ=20.
**Figure 9****:** Distribution of the BGN expression compared to the number of blasts within the bone marrow and the cytogenetic score (CCSS) of the patients. Patients' BGN expression is separated into normal (within heathy control maximum <20), intermediate (<=40) and high (>40).

### DETAILED DESCRIPTION OF THE INVENTION

Unless defined otherwise, technical and scientific terms used herein have the same meaning as commonly understood by one of ordinary skill in the art to which this invention belongs.

The invention is based on the surprising identification of small leucine-rich proteoglycan(s) as a biomarker for a cancer therapy, particularly a cancer immunotherapy.

Hence, a sample obtained from a subject suffering from cancer can be analysed *in vitro* to determine whether cells, particularly MDS/AML cancer blasts, comprised in the sample are indicative of a need of said subject to receive a cancer therapy, particularly a cancer immunotherapy. Thus, herein provided are means and methods for identifying whether a subject suffering from cancer, particularly hematopoietic cancer, is in need to receive a cancer therapy, particularly an immunotherapy.

Particularly, the present invention is based on the surprising and unexpected finding that in a sample of a subject suffering from cancer, particularly hematopoietic cancer, the level of a small leucine-rich proteoglycan (SLRP), preferably biglycan (BGN), or a fragment thereof is indicative of a need of said subject to receive a cancer therapy, particularly a cancer immunotherapy.

In other words the inventors have identified that subjects suffering from cancer, particularly hematopoietic cancer, and in need to receive a cancer therapy, particularly a cancer immunotherapy, have a higher level of a small leucine-rich proteoglycan (SLRP), preferably biglycan (BGN), or a fragment thereof as control samples obtained from subjects not suffering from cancer, particularly hematopoietic cancer.

Determination of the level of a small leucine-rich proteoglycan (SLRP), preferably biglycan (BGN), or a fragment thereof indicative of a subject to suffer from cancer and a need of said subject to receive a cancer therapy, particularly a cancer immunotherapy may, for example, be conducted via determining a certain threshold. Such a threshold may be dependent on the actual assay used. As another example, the level of a small leucine-rich proteoglycan (SLRP), preferably biglycan (BGN), or a fragment thereof may be determined through the comparison of the level of a small leucine-rich proteoglycan (SLRP), preferably biglycan (BGN), or a fragment thereof in a sample of a subject to be identified to a reference level. Such reference level may for example be a level obtained in samples of subjects not suffering from cancer, particularly hematopoietic cancer. Hence, the present invention also relates to determining the level of a small leucine-rich proteoglycan (SLRP), preferably biglycan (BGN), or a fragment thereof in a sample of the subject to be identified, and comparing the determined level to a reference level. Said reference level may be obtained from a sample of at least one healthy subject, preferably a healthy population. More particularly, an increased level of SLRP, preferably BGN, or a fragment thereof in a sample from the subject to be identified compared to a reference level is indicative a subject to suffer from or being at risk of developing cancer and/or of a need of said subject to receive a cancer therapy, particularly a cancer immunotherapy. It is to be understood by those of ordinary skills in the art, that if a preferred SLRP or fragment thereof is chosen to be determined in a sample of a subject to be identified, the reference level should be derived by determining the same preferred SLRP or fragment thereof. Likewise, it is to be understood by those of ordinary skills in the art, that if a preferred cancer is concerned in the context of the invention, the reference level should be derived from subjects without said preferred cancer, e.g. if a subject to be identified suffers from MDS/AML, the reference level shall be derived from subjects without MDS/AML.

Determination of the level of a small leucine-rich proteoglycan (SLRP), preferably biglycan (BGN), or a fragment thereof as described herein can in principle be determined at mRNA and/or protein level. When determined at mRNA level, the expression level may e.g. be determined in a hybridization-based method, a PCR based method, a microarray-based method, a sequencing and/or next generation sequencing method as described also herein below. When determined at protein level, immunoassay methods are typically used, e.g. an immunohistochemistry (IHC) assay as also described herein below.

### DETAILED DEFINITIONS

Hematopoietic cancers are diseases commonly associated with chromosomal abnormalities such as translocations. As used herein, the term "disease" is defined as a deviation from the normal structure or function of any part, organ or system of the body (or any combination thereof). A specific disease is manifested by characteristic symptoms and signs, including both chemical and physical changes. Certain characteristic signs, symptoms, and related factors of the disease can be quantitated through a variety of methods to yield important diagnostic information. As used herein, the term "cancer" refers to uncontrolled cellular growth, and is not limited to any stage, grade, histomorphological feature, invasiveness, aggressivity, or malignancy of an affected tissue or cell aggregation. Thus, as used herein, the term "cancer" also refers to a neoplastic disease, including a tumor mass. As used herein, the term "tumor" refers to all neoplastic cell growth and proliferation, whether malignant or benign, and all pre-cancerous and cancerous cells and tissues. As used herein, the term "neoplastic lesion" or "neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin). The neoplastic disease can be an early, non-metastatic neoplastic disease or a recurrent and/or metastatic neoplastic disease. As used herein, the term "recurrent" refers in particular to the occurrence of metastasis. Such metastasis may be distal metastasis that can appear after the initial diagnosis, even after many years, and therapy of a tumor, to local events such as infiltration of tumor cells into regional lymph nodes, or occurrence of tumor cells at the same site and organ of origin.

As mentioned herein above, in a preferred embodiment, the cancer may be hematopoietic cancer. The terms "hematopoietic cancer", "hematopoietic malignancy" and "hematopoietic neoplasm" are used interchangeably and, as used herein, refer to cancers that affect blood and bone marrow. Hematopoietic cancer may originate from a hematopoietic cell of any lineage, including the lymphoid, myeloid and erythroid lineages. The commonest forms are the various types of leukemia, of lymphoma, and of the progressive, life-threatening forms of the myelodysplastic syndromes. Usually, hematopoietic cancers do not form solid cancers and thus refer to non-solid cancers.

Hematopoietic neoplasms are exemplified by but not limited to leukemia, lymphoma, multiple myeloma, myelodysplastic syndrome (MDS), myeloproliferative disease, and amyloid disease. The term "leukemia" refers to a progressive, malignant disease of the blood or bone marrow, characterized by abnormal proliferation and development of blood cells, usually white blood cells (leukocytes). Leukemias are classified according to the degree of cell differentiation as acute or chronic, and according to predominant type of cell involved as myelogenous or lymphatic. Leukemias are exemplified by acute lymphocytic leukemia (ALL), chronic myelogenous leukemia (CML), acute myeloid leukemia (AML), chronic lymphocytic leukemia (CLL), hairy cell leukemia, etc. The term "lymphoma" refers to a malignant growth of B or T cells in the lymphatic system, presenting as an enlargement of the node (a tumor). The term "lymphoma" includes numerous types of malignant growths, including Hodgkin's lymphoma and non-Hodgkin lymphoma (NHL). "Hodgkin lymphomas" are exemplified by classical Hodgkin lymphomas and nodular lymphocyte-predominant Hodgkin lymphoma. The term "non-Hodgkin lymphoma" refers to a malignant growth of B or T cells in the lymphatic system that is not otherwise classified as a Hodgkin's lymphoma (e.g., having Reed-Sternberg cells in the cancerous area). For example, Non-Hodgkin lymphomas include mature B cell neoplasms and T cell and natural killer cell neoplasms. Mature B cell neoplasms are exemplified by diffuse large B-cell lymphoma (DLBCL), B-cell prolymphocytic leukemia, lymphoplasmacytic lymphoma (such as Waldenström macroglobulinemia), splenic marginal zone lymphoma, plasma cell neoplasms, extranodal marginal zone B cell lymphoma (also called MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, and Burkitt lymphoma/leukemia. Mature T cell and natural killer (NK) cell neoplasms are exemplified by T cell prolymphocytic leukemia, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides/Sezary syndrome, primary cutaneous CD30-positive T cell lymphoproliferative disorders, angioimmunoblastic T cell lymphoma, peripheral T cell lymphoma, unspecified, and anaplastic large cell lymphoma.

Preferably, the hematopoietic cancer as used herein may be selected from one or more of myelodysplastic syndrome (MDS), acute myeloid leukemia (AML), particularly secondary acute myeloid leukemia (sAML), acute lymphoid leukemia (ALL), chronic lymphocytic leukemia (CLL), acute leukemia, B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, non-Hodgkin's lymphoma, Hodgkin's lymphoma, plasmablastic lymphoma, plasmacytoid dendritic cell neoplasm, and Waldenstrom macroglobulinemia. More preferably, the hematopoietic cancer as used herein may be selected from one or more of myelodysplastic syndromes (MDS) and acute myeloid leukemia (AML), particularly secondary acute myeloid leukemia (sAML) chronic myeloid leukemia (CML), acute lymphocytic leukemia (ALL) and chronic lymphocytic leukemia (CLL). Even more preferably, the hematopoietic cancer as used herein may is MDS or AML, particularly sAML.

As used herein, the term "myelodysplastic syndromes" or "MDS" refers to a heterogeneous group of clonal hematopoietic stem cell disorders characterized by ineffective hematopoiesis (blood cell production) involving one or more cell lineages (red blood cells, white blood cells or platelets) and a variable risk of transformation to acute myeloid leukemia (AML).

As used herein, the term "AML" is to be understood to encompass all forms of acute myeloid leukemia and related neoplasms such as according to the French-American-British (FAB) classification system or the World Health Organization (WHO) classification of myeloid neoplasms and acute leukemia and as described herein. Acute myeloid leukemia (AML) can be observed in patients of all ages, but is characteristically associated with increased age. Moreover, several risk factors contributing to the development of secondary AML (sAML) have been identified, such as pre-existing myelodysplastic syndrome (MDS).

The mode of therapy of AML can be determined by the French-American-British (FAB) classification system, in which AML can be divided into subtypes M0 to M7 based on the type of cell from which the leukemia developed and its degree of maturity. Moreover, AML may be diagnosed and the mode of therapy can be determined according to the morphological appearance of blasts in the blood and bone marrow as well as prognostic factors such as cytogenetics and the presence of certain molecular markers.

To illustrate, the analysis of the morphology and cytochemistry of bone marrow blasts and peripheral blood cells can be commonly used to establish a diagnosis. In addition to the diagnosis by cytomorphology alone, diagnosis of AML can be combined with genetic analysis based on, e.g., chromosome analysis, fluorescence in situ hybridization (FISH), or reverse transcription PCR (RT-PCR) and immunophenotyping is also generally used to accurately diagnose AML. In this context, the WHO classification incorporates genetic abnormalities into diagnostic algorithms for the diagnosis of AML. These are (i) acute myeloid leukemia with recurrent genetic abnormalities: AML with t(8;21)(q22;q22); RUNX1-RUNX1T1; AML with inv(16)(p13.1q22) or t(16;16)(p13.1;q22); CBFB-MYH 1; AML with t(9;11)(p22;q23); MLLT3-MLL; AML with t(6;9)(p23;q34); DEK-NUP214; AMLwith inv(3)(q21q26.2) or t(3;3)(q21;q26.2); RPN1-EVI1; AML (megakaryoblastic) with t(1;22)(p13;q13); RBM15-MKL1; Provisional entity: AML with mutated NPM1; Provisional entity: AML with mutated CEBPA; (ii) acute myeloid leukemia with myelodysplasia-related changes; (iii) therapy-related myeloid neoplasms; (iv) acute myeloid leukemia, not otherwise specified: AML with minimal differentiation; AML without maturation; AML with maturation; acute myelomonocytic leukemia; acute monoblastic/monocytic leukemia; acute erythroid leukemia (pure erythroid leukemia; erythroleukemia, erythroid/myeloid); acute megakaryoblastic leukemia; acute basophilic leukemia; acute panmyelosis with myelofibrosis; (v) myeloid sarcoma; (vi) myeloid proliferations related to down syndrome: transient abnormal myelopoiesis; myeloid leukemia associated with down syndrome and (vii) blastic plasmacytoid dendritic cell neoplasm.

A combination of methods cytomorphology (e.g. according to the FAB classification system) and genetic analysis (e.g., according to the WHO) can typically be used to diagnose AML and subtypes thereof.

Currently, most AML patients are treated with chemotherapy such as Cytarabine and afterwards assigned to a post-remission strategy. However, outcomes remain heterogeneous. Moreover, it is difficult for most patients, especially for the older individuals to tolerate chemotherapy. Hence, age remains an important factor for the prediction of therapeutic success. In addition, without further therapy, it can be expected that also younger AML patients, e.g., younger than 60 years, can experience relapse following initial remission.

To address these drawbacks, the development of safe and efficient, targeted approaches for guiding clinical decisions in the treatment of cancer, particularly hematopoietic cancer, are essential.

Provided herein are means and methods for identifying the need of a subject suffering from cancer, particularly hematopoietic cancer, to receive a cancer therapy, particularly a cancer immunotherapy.

The terms "cancer therapy", "cancer treatment" or "treating cancer" and grammatical variations thereof refer to subjecting an individual subject to a protocol, regimen, process or remedy, in which it is desired to obtain a physiologic response or outcome in that subject, e.g., a patient. In particular, the methods and compositions of the present invention may be used to slow the development of disease symptoms or delay the onset of the disease or condition, or halt the progression of disease development. However, because every treated subject may not respond to a particular treatment protocol, regimen, process or remedy, treating does not require that the desired physiologic response or outcome be achieved in each and every subject or subject population, e.g., patient population. Accordingly, a given subject or subject population, e.g., patient population may fail to respond or respond inadequately to treatment. For example, in the context of the present invention, azacytidine may be used as a cancer therapy as described herein and illustrated in the appended examples.

Preferably, the cancer therapy as used herein and in the context of the invention is a cancer immunotherapy. As used herein, the term "cancer immunotherapy" refers to a treatment that uses the body's immune system, either directly or indirectly, to shrink or eradicate cancer. For example, the cancer immunotherapy may stimulate the immune system to treat cancer by improving on the system's natural ability to fight cancer by stimulating the body's own immune system by general means in order to boost the immune system to attack cancer cells. As another example, the cancer immunotherapy may exploit tumor antigens, i.e. the surface molecules of cancer cells such as proteins or other macromolecules and train the immune system to attack cancer cells by targeting the tumor antigens. The cancer immunotherapy as used herein may be selected from the group consisting of chimeric antigen receptor (CAR) T or NK cell therapy, chemotherapy, stem cell transplantation, antibody-based therapy, checkpoint inhibition therapy and vaccines approaches. Preferably, the cancer immunotherapy as used herein may be selected from the group consisting of chimeric antigen receptor (CAR) T-Cell therapy, chemotherapy and stem cell transplantation.

As used herein, the term "CAR T-cell therapy" or "chimeric antigen receptor T-cell therapy" refers to a type of treatment in which T-cells in a subject are changed *ex vivo* in such a manner so that they will attack cancer cells *in vivo* and/or trigger other parts of the immune system to destroy cancer cells. Such T-cells may be, for example, taken from blood of the subject and a gene for a special receptor that binds to a certain protein on the subject's cancer cell is added *ex vivo.* The special receptor may be a man-made receptor and is called a chimeric antigen receptor (CAR). The subject's own T-cells are used to make the CAR T-cells. The CAR T-cells may be grown *ex vivo* and returned to the subject, for example by infusion. The CAR T-cells may be able to identify specific cancer cell antigens. Since different cancer cells may have different antigens, each CAR may be made for a specific cancer antigen. For example, certain kinds of leukemia or lymphoma will have an antigen on the outside of the cancer cells called CD19. The CAR T-cell therapies to treat those cancers are made to connect to the CD-19 antigen and will not work for a cancer that does not have the CD19 antigen. Methods of producing CAR T-cells are well known in the art.

The term "chemotherapy" refers to the administration of chemical agents that inhibit the growth, proliferation and/or survival of cancer cells. Such chemical agents are often directed to intracellular processes necessary for cell growth or division, and are thus particularly effective against cancerous cells, which generally grow and divide rapidly. In general, chemotherapy can include any chemical agent that inhibits, or is designed to inhibit, a cancerous cell or a cell likely to become cancerous.

As used herein, "hematopoietic cell transplantation" refers to the transplantation of blood stem cells derived from the bone marrow or blood of the donor. Transplantation of allogeneic or autologous hematopoietic stem cells is an established treatment for a variety of diseases including hematological cancers, metabolic disorders, and certain genetic diseases. Particularly, hematopoietic stem cells are present in bone marrow, umbilical cord blood, and peripheral blood as described herein below. Thus, hematopoietic stem cells, bone marrow, and umbilical cord blood isolated from peripheral blood can be used as grafts for hematopoietic stem cell transplantation. Hematopoietic stem cell transplantation can be performed to restore hematopoietic function by transplanting hematopoietic stem cells to patients with diseases in which hematopoietic function declines.

The person skilled in the art knows traditional and/or conventional monoclonal antibodies that are used in cancer treatment. Such traditional and/or conventional monoclonal antibodies that are encompassed by the cancer immunotherapy as provided herein include but are not limited to those targeting antigens such as CD20, CD30, CD33, CD123, CD32, CD25, CD44, CD96, CLL-1, and TIM-3; see, e.g. Masarova et al., Immunotherapy, pp. 97-116; 2018.

As used herein, "checkpoint inhibition therapy" refers to a type of treatment in which immune checkpoint inhibitors that modulate cancer immunity are to be administered to a patient. The immune checkpoints are key regulators of the immune system that stimulate or inhibit its actions, which tumors can use to protect themselves from attacks by the immune system. Thus, immune checkpoint inhibitors are a type of drugs that block certain proteins made by some types of immune system cells, such as T cells, and some cancer cells. Hence, immune checkpoint inhibitors can block the inhibitory checkpoints, the so called "brakes" of the immune system, thereby releasing the "brakes" and restoring the immune system function, so that T cells are able to kill cancer cells better. Examples of checkpoint proteins found on T cells or cancer cells include PD-1/PD-L1 and CTLA-4/B7-1/B7-2.

As used herein, the term "cancer vaccine" refers to a type of treatment in which the immune system's ability to recognize and destroy cancer antigens is boosted. Such cancer vaccines may comprise traditional vaccines that target the viruses that can cause certain cancers and may protect against these cancers, however they may not target the cancer cells directly. As such, strains of the human papilloma virus (HPV) have been linked to cervical, anal, throat, and some other cancers. Further, people who have chronic or long-term infections with the hepatitis B virus (HBV) may be at higher risk for liver cancer. Therefore, administration of a vaccine preventing HBV infection may also lower the risk of developing liver cancer. Moreover, cancer vaccines of the present invention may comprise vaccines for treating an existing cancer. For example, cancer vaccines may be produced by immunizing subjects against specific cancer antigens and thereby stimulate the immune system to attack and destroy the cancer cells. In a preferred embodiment of the present invention, the cancer vaccine is a cancer vaccine for treating an existing cancer. Several different types of cancer vaccines are investigated in clinical trials and studies including but not limited to tumor cell vaccines, antigen vaccines, dendritic cell vaccines, vector-based vaccines. Tumor cell vaccines may be made from actual cancer cells that have been removed from the subject during surgery. The cells may be modified (and killed) in the laboratory to increase the probability for them to become attacked by the immune system after they have been injected back into the subject. The subject's immune system may then attack these cells and any similar cells still in the body. Antigen vaccines may boost the immune system by using only one or a few antigen(s), rather than whole tumor cells. The antigens are for example proteins or peptides. Dendritic cell vaccines may be made from the person in whom they will be used and break down cancer cells into antigens that are presented by T cells which may start an immune reaction against any cells in the body that contain these antigens. Vector based vaccines may use special delivery systems (called vectors) to make them more effective. Such vectors may include but are not limited to viruses, bacteria, yeast cells, or other structures that can be used to effectively deliver antigens into the body. In general, types of cancer in which cancer vaccines are now being studied include, for example, brain tumors (especially glioblastoma), breast cancer, cervical cancer, colorectal cancer, kidney cancer, lung cancer, lymphoma, melanoma, pancreas cancer and prostate cancer.

The inventors identified biomarkers that can rapidly and accurately identify patients which will benefit from an immunotherapy. The term "marker" or "biomarker" as used herein refers to a gene, such as mRNA, or protein whose expression in a sample derived from a cell or mammal is altered or modulated, for example, up- or downregulated. Biomarkers can be analysed, e.g., from a sample isolated from the body, to identify the most valuable predictive markers. For example, RNA may be isolated from core biopsies for the gene expression analysis. Based on the expression level data, which may be compared to a reference level, the therapeutic response may be directly evaluated.

As illustrated in the appended examples, the inventors surprisingly identified that a small leucin rich proteoglycans (SLRPs), namely biglycan (BGN), is upregulated in bone marrow samples from MDS and AML patients in need of a cancer therapy, particularly a cancer immunotherapy. Thus, provided herein is the use of SLRP, particularly BGN, or a fragment thereof as a biomarker, wherein the level of SLRP, particularly BGN, of a fragment thereof is determined.

Small leucin rich proteoglycan (SLRP) and biglycans (BGN) are proteoglycans. Small leucin rich proteoglycans (SLRP) comprise proteoglycans of the extracellular matrix (ECM) (Hocking, A. M., et al. Matrix Biol. 17, 1-19 (1998); Lozzo R V. J. Biol. Chem 274, 18843-18846 (1999)). Typically, SLRPs are characterized by a central domain containing leucine rich repeats (LRR) flanked at either side by small cysteine clusters. Examples of SLRPs are described, e.g., in Lozzo et al. (1998) Ann. Rev. Biochem. 67:609, specifically incorporated herein by reference.

SLRP protein cores range from about 35-45 kD with one or two GAG chains attached at the N-terminus. The general structure of the SLRP protein core consists of a tandem array of 6-10 leucine-rich repeats (LRR) flanked by domains with conserved, disulfide-bonded cysteines. Depending upon the extent of glycosylation and number of GAG chains, the native molecular weight ranges from about.100-250 kD. On the basis of their sequence homology, Lozzo et al (1999), *supra,* has proposed that SLRPs be grouped into three classes consisting of: 1) biglycan and decorin; 2) fibromodulin, lumican, keratocan, PREPLP, and osteoadherin; and 3) epiphycan and osteoglycin. The LRRs of the SLRPs mediate protein-protein interactions in a wide variety of intracellular, transmembrane, and extracellular contexts (Kobe & Deisenhofer, (1994) Trends Biochem. Sci. 19: 415-21). The repeats are thought to have a general structure of an alpha-helix followed by beta-sheet in an anti-parallel array, although sequence analysis has suggested that this order might be reversed in the SLRPs (Hocking et al, (1998) Matrix Biol. 17: 1-19). It is likely that the conserved residues of each repeat dictate their secondary structure, while the intervening amino acids determine specificity of ligand binding.

For example, SLRPs as used in the context of the present invention may be encoded by nucleotide sequences which are at least 70%, preferably at least 80%, more preferably at least 85%, even more preferably at least 90%, even more preferably 95%, even more preferably 98%, even more preferably 99% identical to the nucleotide sequence of a SLRP, such as biglycan (BGN) or a fragment thereof. As another example, SLRPs as used in the context of the present invention may have amino acid sequences which are at least 70%, preferably at least 80%, more preferably at least 85%, even more preferably at least 90%, even more preferably 95%, even more preferably 98%, even more preferably 99% identical to the amino acid sequences of a SLRP, such as biglycan or a fragment thereof.

Nucleotide and amino acid sequences of biglycan genes and proteins are provided herein and are also publically available, such as in GenBank. For example, human biglycan can be found under GenBank Accession No. J04599 (human hPGI encoding bone small proteoglycan I (biglycan), described in Fisher et al. (1989) J. Biol. Chem. 264: 4571) and M65154; cow biglycan can be found under GenBank Accession No. L07953; rat biglycan can be found under GenBank Accession No. U17834, mouse biglycan can be found under GenBank Accession No. L20276 and X53928. The biglycan gene is on the X chromosome and is dysregulated in Turner (XO) and Kleinfelter's Syndromes diseases.

In a preferred embodiment of the invention, SLRP, preferably BGN, or a fragment thereof is expressed on the cell surface, preferably on the cell surface of hematopoietic cells. Particularly, SLPPs for use in the present invention include class I SLRPs, such as biglycan and/or decorin or a fragment thereof. Biglycan (BGN) is an extracellular matrix protein and a member of the small leucine rich proteoglycan (SLRP) family, which is characterized by the presence of repeated leucine rich amino acid motif (Fisher et al. J. Biol. Chem. 264, 4571-4576. 1989).

For example, biglycan (BGN) as described herein refers to human biglycan (GenBank Accession No. NP001702 and Fisher et al, *supra*). For example, biclycan may refer to human biglycan comprising SEQ ID NO. 1 Human biglycan may contain 368 amino acids, of which amino acids 1-19 constitute a signal peptide. Thus, biglycan without a signal peptide consists of amino acids 20-368. The mature biglycan protein consists of amino acids 38-368, since amino acids 1-37, being a pre-propeptide, are cleaved during processing. Amino acids 38-80 correspond to the N-terminal cysteine-rich region. About amino acids 81-314 corresponds to the leucine rich repeat region, containing 10 repeats of about 24 or 23 amino acids. Preferred biglycans as used herein include human biglycan and fragments thereof. For example, biglycans as used herein include human biglycan comprising SEQ ID NO. 1. Biglycans as used herein may also include splice variants such as human biglycan comprising SEQ ID NO. 2.

Typically, BGN comprises a protein core containing leucine-rich repeats (LRRs), to which one or two glycosaminoglycan (GAG) side chains can be covalently bound. Their composition is tissue specific and can be regulated at a number of levels (Hocking et al, (1998) Matrix Biol 17: 1-19). For example, biglycan may be expressed as a proteoglycan or as a mature form lacking GAG side chains ("nonglycanated"). As another example, the biglycan from skin and cartilage is typically bound to GAG which is predominantly dermatan sulfate, while biglycan synthesized in bone is a chondroitin sulfate proteoglycan. Both the protein core and the cell type contribute to the distinct glycosylation of biglycans.

Biglycans have been shown to be expressed on cell surface or as an extracellular matrix component particularly of the skeleton including bone, cartilage, tendon, teeth and muscle (Bianco P., Fisher, et al. J. Histochem. Cytochem. 38, 1549-1568 (1990); Wadhwa Set al. Crit Rev Eukaryot Gene Expr. 2004;14(4):301-315.). Functions of biglycan are dependent on their site of expression. For example, in the cardiovascular system, biglycan may play a crucial role in atherosclerosis Fedarko, et al.. J. Biol. Chem. 265, 12200-12209 (1990); Klezovitch, et al. Trends Cardiovasc. Med. 11, 263-268 (2001).

Biglycan as described herein also refers to proteoglycans, proteins or peptides having at least one biological activity of human or other animal originated biglycan. A biological activity of a biglycan can, for example, refer to the ability to maintain the integrity of a plasma membrane.

Biglycans as described herein are not limited to full length biglycan, but also include portions or fragments thereof having at least one activity of biglycan. For example, such a fragment may include but not be limited to decorin. Decorin is a matrix proteoglycan belonging to the small leucine rich repeat (LRR) proteoglycans family. It is mainly secreted by fibroblasts. Its normal physiological function is to fine tune the collagen fibrinogenesis. It has several binding domains for other matrix molecules such as fibronectin, thrombospondin, collagen, and growth factor TGF-beta, EGF receptor, as well as several metal ions.

In the context of the present invention, the level of SLRP, preferably BGN, or a fragment thereof is determined in a sample of a subject. As used herein, the terms "sample" or "biological sample" as are used interchangeably and refer to a sample obtained from the subject. The sample may be of any diseased tissue or fluid comprising tumor cells suitable for carrying out the method of the present invention, i.e. for identifying the need of a subject suffering from cancer to receive a cancer therapy, particularly a cancer immunotherapy. However, once the subject is identified to be in need of a cancer therapy, particularly a cancer immunotherapy, according to the methods of the present invention, the subject will receive the cancer therapy, particularly the cancer immunotherapy, as soon as possible. In particular, the sample may be obtained from any diseased tissue and/or fluid comprising tumor cells of a subject suffering from cancer. The sample may also be a bodily fluid with integrated cancer cells. Such fluids may include the lymph and/or ascites. Preferably, the tissue and/or fluid of the sample may be taken from any material of the cancer and/or from any material associated with the cancer. Such a sample may, for example, comprise cells obtained from the subject. In one embodiment, the sample has to be a cancer sample. A "cancer sample" is a biological sample containing cancer cells, whether intact or degraded. In one embodiment, the sample is a cancer sample obtained from said subject.

The sample may also include sections of tissues. Such sections of tissues encompass frozen or fixed sections. These frozen or fixed sections may be used, e.g. for histological purposes. In one embodiment, the sample from said subject is a formalin-fixed paraffin embedded sample or a fresh-frozen sample.

A sample to be analyzed may be taken by aspiration or punctuation, excision or by any other surgical method leading to biopsy or resected cellular material.

Particularly, the sample may be a blood sample, a fluid bone marrow sample, a sample of lymphoid tissue, or a sample from other materials containing neoplastic hematopoietic cells. Preferably, the sample may be selected from the group consisting of a blood sample, preferably a peripheral blood sample and serum, and a sample from bone marrow. "Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain. It is to be understood by a person skilled in the art that a sample as used herein and in the context of the present invention may be a sample comprising hematopoietic stem cells.

Hematopoietic stem cells refer to a subset of multipotent stem cells that give rise to all the blood or immune cell types, including myeloid (monocytes and macrophages, neutrophils, basophils, eosinophils, erythrocytes, megakaryocytes/platelets, dendritic cells), and lymphoid lineages (T -cells, B-cells, NKT -cells, NK-cells). "Stem cells," as used herein, refer to cells that retain the ability to renew themselves through mitotic cell division and can differentiate into a diverse range of specialized cell types. The two broad types of mammalian stem cells are: embryonic stem (ES) cells that are found in blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues. Pluripotent stem cells can differentiate into cells derived from any of the three germ layers.

Accordingly, "hematopoietic stem cells," or "HSCs," as the terms are used herein, encompass all multipotent cells capable of differentiating into all the cell types of the hematopoietic system, including, but not limited to, granulocytes, monocytes, erythrocytes, megakaryocytes, B-cells and T-cells, and having multi-lineage hematopoietic differentiation potential and sustained self- renewal activity. "Self-renewal" refers to the ability of a cell to divide and generate at least one daughter cell with the identical (e.g., self-renewing) characteristics of the parent cell. The second daughter cell may commit to a particular differentiation pathway. For example, a self-renewing hematopoietic stem cell divides and forms one daughter stem cell and another daughter cell committed to differentiation in the myeloid or lymphoid pathway. In contrast, a committed progenitor cell has typically lost the self-renewal capacity, and upon cell division produces two daughter cells that display a more differentiated (i.e., restricted) phenotype.

As provided herein and in the context of the present invention, the level of SLRP, preferably BGN, or a fragment thereof is determined. The term "determination" or "determining" is used herein to refer to the evaluation of the need of a patient to receive a therapy, particularly an immunotherapy and/or the diagnosis, prognosis, risk assessment, risk stratification, therapy control and/or post-operative control of cancer in a subject. Particularly, gene or protein expression levels may be determined. The term "level" as used herein refers to the quantity of the molecular entity of the marker (i.e. SLRP ,particularly BGN, or a fragment thereof) in a sample that is obtained from the subject. In other words, the concentration of the marker is determined in the sample. It is also envisaged that a fragment of the marker can be detected and quantified. Thus, it is apparent to the person skilled in the art, in order to determine the level of a marker, parts and fragments of said marker can be used instead. Suitable method to determine the level of a marker are described herein below in detail. As used herein, the term "marker" relates to measurable and quantifiable biological markers which serve as indices for health- and physiology-related assessments, such as a disease/disorder/clinical condition risk. Furthermore, a marker is defined as a characteristic that is objectively measured and evaluated as an indicator of normal biological processes, pathogenic processes, or pharmacologic responses to a therapeutic intervention. As discussed herein above a biomarker may be measured on a biological sample.

In one embodiment, the level of a marker (i.e. SLRP, particularly BGN, or a fragment thereof) is the protein expression level or the RNA expression level, preferably mRNA expression level. For example, the expression level refers to a determined level of gene expression. A "gene" is a set of segments of nucleic acid that contains the information necessary to produce a functional RNA product. A "gene product" is a biological molecule produced through transcription or expression of a gene, e.g., an mRNA, cDNA or the translated protein. An "mRNA" is the transcribed product of a gene and shall have the ordinary meaning understood by a person skilled in the art. A "molecule derived from a mRNA" is a molecule which is chemically or enzymatically obtained from a mRNA template, such as cDNA. The expression level may be a determined level of protein, RNA, or mRNA expression as an absolute value or compared to a reference level, such as to a reference gene, to the average of two or more reference levels, or to a computed average expression value or to another informative protein, RNA or mRNA without the use of a reference sample.

In one embodiment of the present invention, the method further comprises comparing the determined level of a SLRP, preferably BGN, or a fragment thereof to a reference level, preferably the reference level comprises the level of SLRP, preferably BGN, or a fragment thereof in a sample of at least one healthy subject, preferably the mean level of the SLRP, preferably BGN, or a fragment thereof in samples obtained from a healthy population. A need for a cancer therapy, particularly a cancer immunotherapy, of a subject suffering from cancer may be identified based on the comparison of the level of SLRP, particularly BGN, or a fragment thereof, with a reference level. Equally, the diagnosis, prognosis, risk assessment, risk stratification, therapy control and/or post-operative control of cancer in a subject may be identified based on the comparison of the level of SLRP, particularly BGN, or a fragment thereof, with a reference level.

Such a "reference level" can be a level as described herein above or below. For example, the reference level may be a numerical cutoff value, which can be derived from a reference measurement of a marker in a sample or in a plurality of samples. For example, one way of determining such cut-off value is to construct a receiver-operator curve (ROC curve) as described herein below on the basis of all conceivable cut-off values, determining the single point on the ROC curve with the lowest proximity to the upper left corner (0/1) in the ROC plot. Typically, most of the time cut-off values will be determined by less formalized procedures by choosing the combination of sensitivity and specify determined by such cut-off value providing the most beneficial medical information to the problem investigated.

The method of the invention may comprise comparing the expression level of a marker (i.e. SLRP, particularly BGN, or a fragment thereof) to a predetermined reference level. As another example, the reference level can be determined from a validation cohort, preferentially as the median or any other percentile, or preferentially by specifying one or more aims for statistical measures such as specificity, sensitivity, negative predictive value, positive predictive value, overall correctness, area under receiver operator curve, odds ratio, hazard ratio, or c-index. The sensitivity and specificity of such statistical measures and tests depend on more than just the analytical "quality" of the tests, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations (i.e. patients suffering from breast cancer). For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, e.g., Hanley et al.1982. Radiology 143: 29-36). Preferably, ROC curves result in an AUC of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In one embodiment of the present invention, an increased level of SLRP, preferably BGN, or a fragment thereof in the sample from the subject to be identified compared to the reference level is indicative of a need of said subject to receive a cancer therapy, particularly a cancer immunotherapy. For example, a reference level may be lower than the level of a SLRP, particularly BGN, or a fragment thereof in a sample from a subject to be identified. For example, the reference level may be 2-fold lower than the level of SLRP, particularly BGN, or a fragment thereof in a sample from a subject to be identified.

The difference between the level of a marker (i.e. SLRP, particularly BGN, or a fragment thereof) compared to the reference level may alternatively be determined by absolute values, e.g. by the difference of the expression level of SLRP, particularly BGN, or a fragment thereof and the reference level, or by relative values, e.g. by the percentage increase or decrease of the level of SLRP, particularly BGN, or a fragment thereof compared to the reference level. The level of SLRP, particularly BGN, or a fragment thereof which deviates from the reference level may be indicative for identifying the need of a subject suffering from or being at risk of developing cancer to receive a cancer therapy, particularly a cancer immunotherapy. Equally, The level of SLRP, particularly BGN, or a fragment thereof which deviates from the reference level may be indicative forthe diagnosis, prognosis, risk assessment, risk stratification, therapy control and/or post-operative control of cancer in a subject to be identified and/or a need of a subject identified to suffer from or being at risk of developing cancer to receive a cancer therapy, particularly a cancer immunotherapy.

In other words, an upregulation of the level of SLRP, particularly BGN, or a fragment thereof, compared to the reference level may be indicative for a response and/or benefit from a cancer therapy, particularly a cancer immunotherapy in a subject. In particular, the extent of upregulation of the level of SLRP, particularly BGN, or a fragment thereof compared to the reference level may be indicative for a response and/or benefit of the cancer therapy in a subject suffering from cancer, in particular hematopoietic cancer. For example, the level of SLRP, particularly BGN, or a fragment thereof above by 3-fold rather than above 2-fold compared to the reference level may be indicative with a higher likelihood for a response and/or benefit from a cancer therapy in said subject.

The skilled artisan will understand that associating a marker, i.e. the level of SLRP, particularly BGN, or a fragment thereof, with the subject's need of receiving a cancer therapy and/or with the diagnosis, prognosis, risk assessment, risk stratification, therapy control, particularly hematopoietic cancer, is a statistical analysis. For example, a marker level of higher than X may signal that a subject is more likely to be in need of a cancer therapy and/or to suffer from cancer than a subject with a level less than or equal to X, as determined by a level of statistical significance. Additionally, a change in marker concentration from baseline levels may be reflective of a subject in need of a cancer therapy and/or to suffer from cancer, and the degree of change in marker level may be related to the severity of the prognosis. Statistical significance is often determined by comparing two or more populations, and determining a confidence interval and/or a p value; see, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. For example, the level of a marker is indicative for a subject in need of a cancer therapy and/or to suffer from cancer compared to the level of a reference level at a p-value equal or below 0.05, preferably 0.01, more preferably 0.001 and even more preferably below 0.0001.

The present invention also relates to the use of the method for identifying the need of a subject suffering from or being at risk of developing cancer to receive a cancer therapy, particularly a cancer immunotherapy. Equally, the present invention relates to the use of the method for the diagnosis, prognosis, risk assessment, risk stratification, therapy control and/or post-operative control of cancer in a subject.

When referring to markers (i.e. SLRP, particularly BGN, or a fragment thereof) of the present invention as identified by the gene names mentioned herein above, the person skilled in the art how to derive the respective RNA, in particular the mRNA, or the protein of the marker identified by its gene name. In this context, the gene names as used in the context of the present invention refer to gene names according to the official gene symbols provided by the HGNC (HUGO Gene Nomenclature Committee) and as used by the NCBI (National Center for Biotechnology Information).

In one embodiment, the expression level is the RNA expression level, preferably mRNA expression level, and is determined by at least one of a hybridization based method, a PCR based method, a microarray based method, a sequencing and/or next generation sequencing approach. The term "a PCR based method" as used herein refers to methods comprising a polymerase chain reaction (PCR). This is a method of exponentially amplifying nucleic acids, e.g. DNA by enzymatic replication in vitro. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). Moreover, PCR-based methods comprise e.g. real time PCR, and, particularly suited for the analysis of expression levels, kinetic or quantitative PCR (qPCR).

The term "Quantitative PCR" (qPCR)" refers to any type of a PCR method which allows the quantification of the template in a sample. Quantitative real-time PCR comprise different techniques of performance or product detection as for example the TaqMan technique or the LightCycler technique. The TaqMan technique, for examples, uses a dual-labelled fluorogenic probe. The TaqMan real-time PCR measures accumulation of a product via the fluorophore during the exponential stages of the PCR, rather than at the end point as in conventional PCR. The exponential increase of the product is used to determine the threshold cycle, CT, e.g., the number of PCR cycles at which a significant exponential increase in fluorescence is detected, and which is directly correlated with the number of copies of DNA template present in the reaction. The set-up of the reaction is very similar to a conventional PCR, but is carried out in a real-time thermal cycler that allows measurement of fluorescent molecules in the PCR tubes. Different from regular PCR, in TaqMan real-time PCR a probe is added to the reaction, e.g., a single-stranded oligonucleotide complementary to a segment of 20-60 nucleotides within the DNA template and located between the two primers. A fluorescent reporter or fluorophore (e.g., 6-carboxyfluorescein, acronym: FAM, or tetrachlorofluorescin, acronym: TET) and quencher (e.g., tetramethylrhodamine, acronym: TAMRA, of dihydrocyclopyrroloindole tripeptide 'black hole quencher', acronym: BHQ) are covalently attached to the 5' and 3' ends of the probe, respectively. The close proximity between fluorophore and quencher attached to the probe inhibits fluorescence from the fluorophore. During PCR, as DNA synthesis commences, the 5' to 3' exonuclease activity of the Taq polymerase degrades that proportion of the probe that has annealed to the template. Degradation of the probe releases the fluorophore from it and breaks the close proximity to the quencher, thus relieving the quenching effect and allowing fluorescence of the fluorophore. Hence, fluorescence detected in the real time PCR thermal cycler is directly proportional to the fluorophore released and the amount of DNA template present in the PCR.

As used herein, the term "hybridization based method" refers to a method, wherein complementary, single-stranded nucleic acids or nucleotide analogues may be combined into a single double stranded molecule. Nucleotides or nucleotide analogues will bind to their complement under normal conditions, so two complementary strands will bind to each other. In bioanalytics, very often labelled, single stranded probes are in order to find complementary target sequences. If such sequences exist in the sample, the probes will hybridize to said sequences which can then be detected due to the label. Other hybridization based methods comprise microarray and/or biochip methods. For example, probes may be immobilized on a solid phase, which is then exposed to a sample. If complementary nucleic acids exist in the sample, these will hybridize to the probes and can thus be detected. These approaches are also known as "array based methods". Yet another hybridization based method is PCR, which is described above. When it comes to the determination of expression levels, hybridization based methods may for example be used to determine the amount of mRNA for a given gene. An oligonucleotide capable of specifically binding sequences a gene or fragments thereof relates to an oligonucleotide which specifically hybridizes to a gene or gene product, such as the gene's mRNA or cDNA or to a fragment thereof. To specifically detect the gene or gene product, it is not necessary to detect the entire gene sequence. A fragment of about 20-150 bases will contain enough sequence specific information to allow specific hybridization.

By "array" or "matrix" an arrangement of addressable locations or "addresses" on a device is meant. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, nucleotide analogues, polynucleotides, polymers of nucleotide analogues, morpholino oligomers or larger portions of genes. The nucleic acid and/or analogue on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm².

In one embodiment, the expression level of a marker (i.e. SLRP, particularly BGN, or a fragment thereof) may be the protein level. It is clear to the person skilled in the art that a reference to a nucleotide sequence may comprise reference to the associated protein sequence which is coded by said nucleotide sequence. The expression level of a protein may be measured indirectly, e.g. by obtaining a signal wherein the signal strength is correlated to the amount of mRNA transcripts of that gene or it may be obtained directly at a protein level, e.g., by immunohistochemistry, CISH, ELISA (enzyme linked immunoassay), RIA (radioimmunoassay), flow cytometry, mass cytometry or the use of protein microarrays, two- hybrid screening blotting methods including Western blot, one- and two dimensional gel electrophoresis, isoelectric focusing as well as methods being based on mass spectrometry like MALDI-TOF. The term "immunohistochemistry" or IHC refers to the process of localizing proteins in cells of a tissue section exploiting the principle of antibodies binding specifically to antigens in biological tissues. Immunohistochemical staining is widely used in the diagnosis and treatment of cancer. Specific molecular markers are characteristic of particular cancer types. IHC is also widely used in basic research to understand the distribution and localization of biomarkers in different parts of a tissue.

For the purposes of the present invention the "subject" (or "patient") may be a mammal. In the context of the present invention, the term "subject" includes both humans and other mammals. Thus, the herein provided methods are applicable to both human and animal subjects, i.e. the method can be used for medical and veterinary purposes. Accordingly, said subject may be an animal such as a mouse, rat, hamster, rabbit, guinea pig, ferret, cat, dog, sheep, bovine species, horse, camel, or primate. Most preferably the subject is human. As described herein, the patient may be a patient suffering from cancer, particularly hematopoietic cancer (e.g., MDS ans/or AML). As another example, the patient as described herein may refer to a patient suffering from or being at risk of developing cancer, particularly hematopoietic cancer (e.g., MDS and/or AML).

As used herein, "identifying a subject in need to receive a cancer therapy" refers to the identification of a subject to respond to and/or benefit from a cancer therapy, particularly a cancer immunotherapy. As used herein, the term "response" refers to any response to a cancer therapy, particularly a cancer immunotherapy, as described herein above and below. As used herein, "benefit" from a given therapy is an improvement in health or wellbeing that can be observed in patients under said therapy, but it is not observed in patients not receiving this therapy. Non-limiting examples commonly used in oncology to gauge a response and/or benefit from therapy are survival, disease free survival, metastasis free survival, disappearance of metastasis, tumor regression, and tumor remission.

"Diagnosis" in the context of the present invention relates to the recognition and detection of a disease or clinical condition in a subject and may also comprise differential diagnosis. Also the assessment of the severity of a disease or clinical condition may in certain embodiments be encompassed by the term "diagnosis".

"Prognosis" relates to the prediction of an outcome or a specific risk for a subject suffering from a particular disease or clinical condition. This may include an estimation of the chance of recovery or the chance of an adverse outcome for said subject.

In the present invention, the term "risk assessment" or "risk stratification" relates to the grouping of subjects into different risk groups according to their further prognosis. Risk stratification also relates to stratification for applying preventive and/or therapeutic measures.

The term "therapy control" in the context of the present invention refers to the monitoring and/or adjustment of a therapeutic treatment of said patient.

The term "post-operative control" in the context of the present invention refers to the monitoring of said patient following a surgical procedure of said patient.

The present invention also relates to kits and the use of kits for identifying the need of a subject suffering from cancer to receive a cancer therapy, particularly a cancer immunotherapy. Equally, the present invention also relates to kits and the use of kits for the diagnosis, prognosis, risk assessment, risk stratification, therapy control and/or post-operative control of cancer in a subject.

The kit may comprise one or more detection reagents for determining the level of SLRP, particularly BGN, or a fragment thereof and reference data including the reference level of SLRP, particularly BGN, or a fragment thereof, optionally wherein said detection reagents comprise at least a pair of oligonucleotides capable of specifically binding to SLRP, particularly BGN, or a fragment thereof. As used herein, the term "primer" refers to the ordinary meaning of this term which is well known to the person skilled in the art of molecular biology. Primers shall be understood as being polynucleotide molecules having a sequence identical, complementary, homologous, or homologous to the complement of the regions of a target molecule, which is to be detected or quantified, e.g. SLRP, particularly BGN, or a fragment thereof.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention.

### Sequences:

Sequence 1 (SEQ ID NO:1): BGN Homo sapiens aa sequence (UniProtKB-P21810
PGS1_HUMAN):








Sequence 2 (SEQ ID NO:2): BGN Homo sapiens computationally mapped potential isoform aa
sequence (UniProtKB C9JKG1_HUMAN):

MWPLWRLVSLLALSQALPFEQRGFWDFTLDDGPFMMNDEEASGADTSGVLDPDSVTPTYS
AMCPFGCHCHLRVVQCSDLGLKSVPKEISPDTTLLDLQNNDISELRKDDFKGLQHLYVRS
WEEPAGLQQRAGVRALVLVNNKISKIHEKAFSPLRKLQKLYISKNHLVEIPPNLPSSLVE
LRIHDNRIRKVPKGVFSGLRNMNCIEMGGNPLENSGFEPGAFDGLKLNYLRISEAKLT

### EXAMPLES

### Example 1:

### Materials and methods

### Patient characteristics

Bone marrow biopsies (BMB) collected between 2014 and 2019 at the Medical Faculty of the Martin-Luther University Halle-Wittenberg, Germany, were part of the routine diagnostic approach based on screening and/or treating patients within clinical trials. The cohort consist of BMB from 102 patients divided in 69 MDS patients with different blast counts (without and with excess of blasts (EB-1-2)) and 33 BMB of sAML patients. All MDS/sAML specimen presented myeloid blasts with a CD34⁺ immunophenotype and their clinico-pathological characteristics are summarized in Table 1. Furthermore, 45 BMB from age-matched patients with normal blood cell count and without evidence of myeloid or lymphoid neoplasia within the BMB served as control samples.

Clinical, laboratory, molecular and cytogenetic data including the CCSS, risk group according the IPSS-R and treatment data were collected from the medical records. MDS patients were stratified into the following disease groups: MDS without EB (<5% blasts), MDS with EB-1 (MDS-EB-1, 5-9.9% blasts), MDS with EB-2 (MDS-EB-2, 10-19.9% blasts) and sAML (≥20% blasts) based on the cytological blast count. sAML patients were further divided in two groups characterized by BM blast counts of 20-29.9% and higher than 30%.

### Study approval

Informed consent was obtained from all patients for the use of their diagnostic material for scientific research, which was approved by the Ethical Committee of the Medical Faculty of the Martin Luther University Halle-Wittenberg, Halle, Germany (2017-81). In addition, this Ethical Committee also approved the scientific use of formalin fixed and paraffin embedded (FFPE) samples.

### Standard evaluation of the bone marrow and BGN immunohistochemistry

Diagnosis of MDS and sAML with myelodysplasia related changes was performed according to the diagnostic criteria of the World Health Organization (WHO) classification of tumors of hematopoietic and lymphoid tissues, 4^{th} edition 2017 (Grimm KE 2019, PMID: 30380402).

To confirm the diagnosis of MDS and sAML, conventional histological and cytological examination as well as immunohistochemistry (IHC) were performed. Monoclonal antibodies (mAb) directed against CD34 (clone QBend/10, Labvision, Germany), CD117 (clone CD117, c-kit A4502, Dako, USA), MPO (clone myeloperoxidase A0398, Dako, USA), lysozyme (EP134, Epitomics, USA) and CD71 (MRQ-48, Cell Marque, USA) were used according to the supplier's instructions. Next to standard evaluation, samples were stained with a BGN-specific mAb as described.

### Multispectral Imaging (MSI)

The frequency, localization and spatial proximity of immune cell subpopulations, CD34⁺ hematopoietic stem and progenitor cell (HSPC) and of CD34⁺ blasts in case of malignancy were analyzed by MSI. The staining procedure was performed as recently described (Bauer M 2020, PMID: 32122554; Bauer M 2021, PMID: 33430322) . The marker panel used for staining included mAb directed against CD34 (QBend, Labvision, Germany, 1:500, pH6), CD3 (Labvision, Germany, clone SP7), CD8 (Abcam, UK, clone SP16), FOXP3 (Abcam, UK, clone 236A/E7) and MUMlp (Dako, USA, cloneMUM1p). Briefly, all primary mAb were incubated for 30 minutes. Tyramide signal amplification (TSA) visualization was performed using the Opal seven-color IHC kit containing fluorophores Opal 540, Opal 570, Opal 620, Opal 650, Opal 690 (PerkinElmer Inc., USA) and DAPI. Stained slides were imaged employing the PerkinElmer Vectra Polaris platform.

### Statistics and software

Statistical analyses were performed employing IBM SPSS Statistics. Kolmogorov-Smirnov test revealed non-parametric data (p<0.05). The Mann-Whitney U test was employed to compare clinical data, frequencies of immune cell subpopulations and their spatial distribution. P values <0.05 were considered statistically significant.

### Results

### Cytogenetic alterations and the frequency and histotopography of the immune cell subsets in MDS and AML

Based on the cytogenetic aberrations in MDS without EB and EB 1-2, patients were classified according to the CCSS and the respective subgroups were compared (Table 1). The lower risk subgroup comprised samples with very low and low risk cytogenetic aberrations (CCSS 1-2), while diseases with intermediate, high, and very high cytogenetic scores were defined as higher risk subgroup (CCSS 3-5). The frequency of CD3⁺FOXP3⁺ T cells and MUM1p⁺CD3⁻ B/plasma cells was significantly higher in CCSS high risk cases compared to CCSS low risk cases (p=0.042; p=0.004). No significant differences in the frequency of CD3⁺CD8⁺ and CD3⁺CD8⁻T cells were detected between CCSS low risk, CCSS high risk and control cases. Furthermore, no significant differences in the frequency of T cell and MUM1p⁺CD3⁻ B/plasma cell subpopulations were detected in patients with normal or abnormal karyotypes.

### BGN expression in AML/MDS

First, the expression of biglycan in five AML and one MDS cell lines was determined. In three out of six AML/MDS cell lines high levels of biglycan protein expression was detected by Western blot analysis, but also on the cell surface by flow cytometry using a biglycan specific antibody. In contrast, low or only marginal surface expression of BGN was found on healthy donor peripheral blood mononuclear cells (PBMC). This was confirmed by further immunohistochemical analysis demonstrating a high frequency of BGN expression in bone marrow (BM) samples from MDS and sAML patients compared to BM samples of patients without hematologic disease. Based on these results, a biglycan score was developed and associated this to clinical pathological characteristics, which comprises on the frequency of blasts as on the CCSS score.

BGN expression was associated with the immune cell infiltration. For this BMB from 45 control samples as well as 69 MDS and 33 sAML patients were analyzed by simultaneous staining for the markers CD3, CD8, FOXP3, MUMlp and CD34, combined with nuclear staining using DAPI and associated to BGN expression (Fig. 1). BGN expressing BM samples had a higher CD8⁺ T cell infiltration compared to BGN negative cells, while the CD4⁺ T cell frequency was lower in BGN⁺ cells. This appears to be directly associated with an increase of immune suppressive FOXp3⁺ cells, which were high in BGN⁺ samples. Furthermore, azacytidine treatment downregulates the frequency of BGN⁺ cells. In order to understand the mechanisms of BGN, the caspase activity was determined of BGN⁻ and BGN⁺ MDS and AML subclones demonstrating that BGN⁺ subclones had a higher caspase activity when they expressed TLR4. The results demonstrate that BGN is highly expressed or the cell surface of bone marrow samples of MDS and AML, while it is only marginally expressed on hematopoeitic cells and bone marrow biopsies of healthy individuals, reflecting that BGN is a target for MDS and AML, particularly sAML.

**Table 1: Clinico-pathological characteristics and sample specifications in terms of risk factors, treatment and prognosis**

| | | **MDS** | **sAML** | **Controls** |
|---|---|---|---|---|
| **number of samples** | | 69 | 33 | 45 |
| **age** | | 68,2 (42-86) | 68,9 (47-82) | 59,6 (39-84) |
| **sex** | male | 39 | 18 | 24 |
| | female | 30 | 15 | 21 |
| **MDS** | without excess of blasts | 22 | - | - |
| | with excess of blasts 1 (EB-1) | 26 | - | - |
| | with excess of blasts 2 (EB-2) | 21 | - | - |
| **CCSS** | **1 -** loss of Y chromosome, del(11q) | 7 | 2 | - |
| | **2** - normal karyotype, del(5q), del(12p), del(20q) | 27 | 5 | - |
| | **3** - del(7q), gain of chromosome 8 and 19, isochromosome 17q | 17 | 3 | - |
| | **4** - gain of chromosome 3 and 7, complex (>2) | 1 | 2 | - |
| | **5** - complex (>3) | 17 | 9 | - |
| **IPSS-R** | **1 -** very low risk | 2 | - | - |
| | **2** - low risk | 6 | - | - |
| | **3** - intermediate risk | 20 | - | - |
| | **4** - high risk | 26 | - | - |
| | **5** - very high risk | 15 | - | - |
| **patients without therapy** (neither allogeneic stem cell transplantation nor HMA treatment) | | 35 | | |
| **patients with prior treatment with HMA** | | 17 | 6 | - |
| **patients with MDS with treatment response to HMA** | | 12 | - | - |
| **patients that received allogeneic stem cell transplantation** | | 23 | 9 | - |
| **MDS with further progress to sAML** | | 18 | - | - |

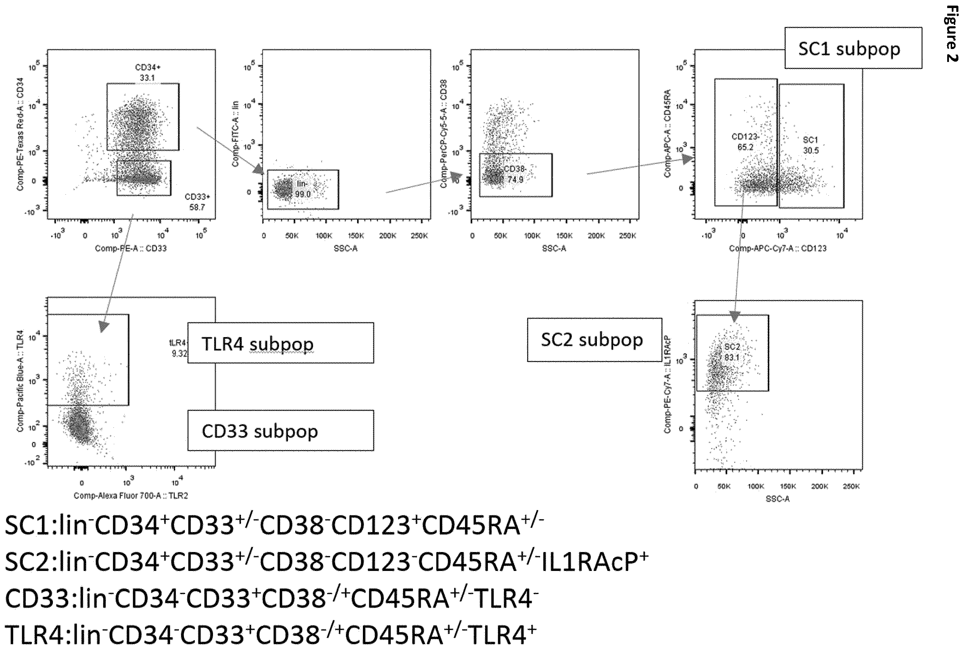

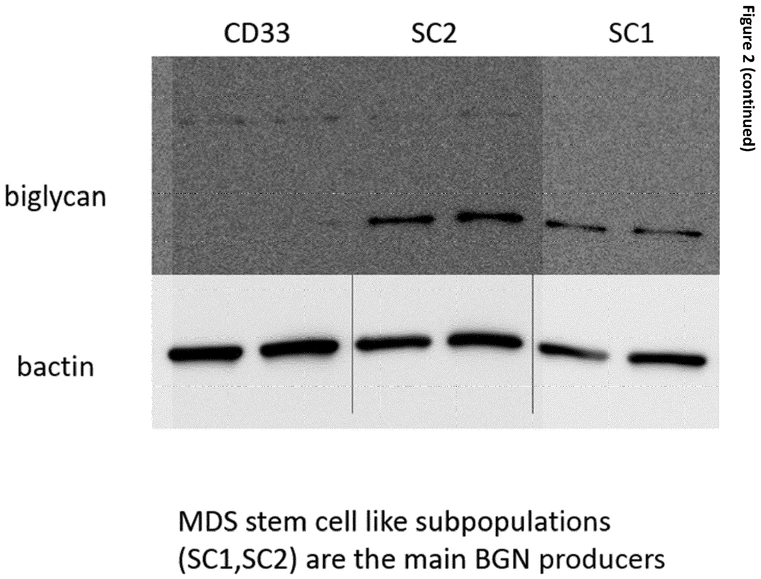

Fig. 2: MDS-L cells were stained with an 8 color panel (CD34- Texas Red, CD33 - PE, lin - FITC, CD38 - PerCP Cy5.5, CD45RA - APC, CD123 - APC-Fire, IL1RAcP - PE vio 770, Zombie aqua) and further analyzed in subpopulations following the gating strategy depicted in Fig 2. Subpopulations were sorted using a FACS Aria Fusion machine and their BGN protein levels were identified through Western blot. SC1 and SC2 subpopulations with a phenotype similar to *in vivo* MDS stem cells (Chen et al., Nat Med. 2019 Jan;25(1):103-110. doi: 10.1038/s41591-018-0267-4. PMID: 30510255) appear to be the major BGN expressing cells.

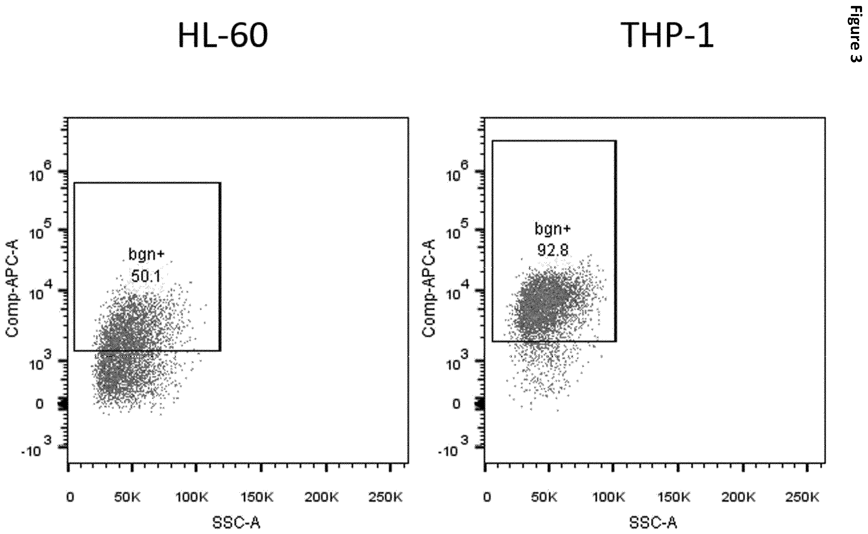

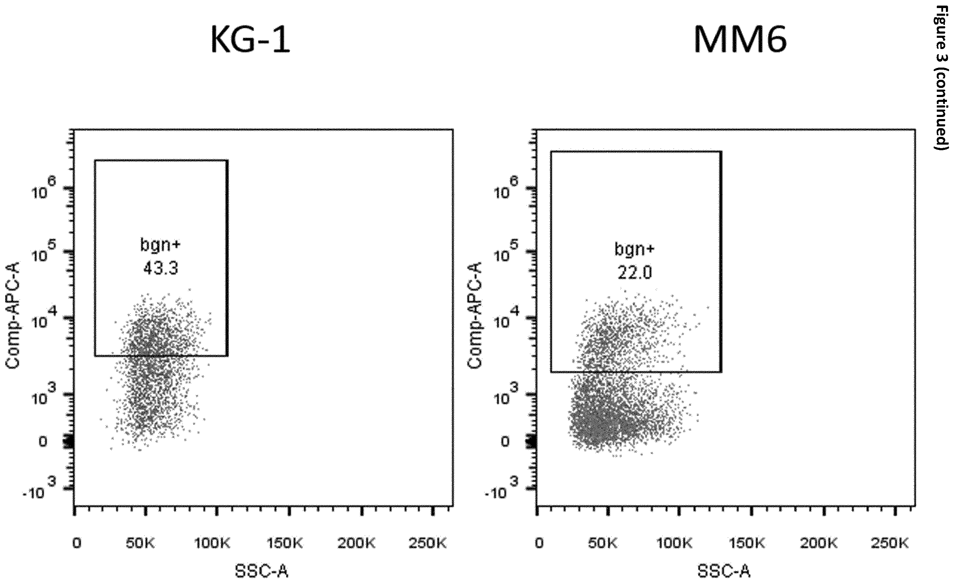

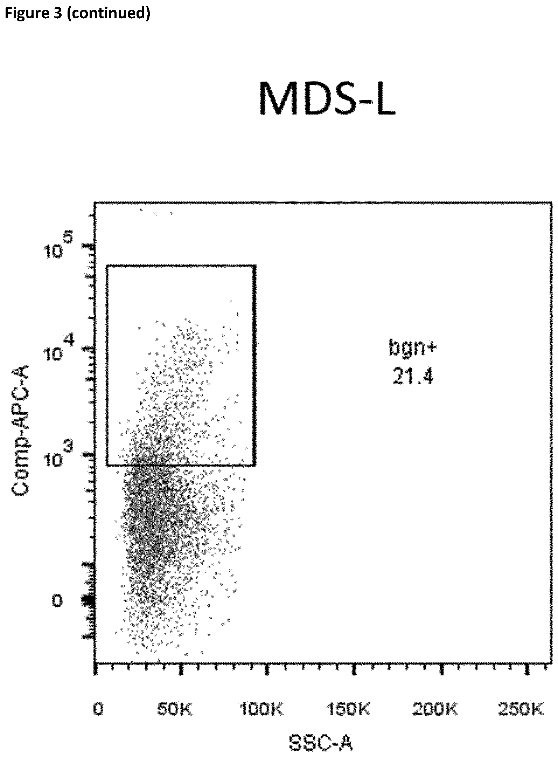

Fig. 3: BGN surface staining of 4 AML and 1 MDS (MDS-L) cell line. Positivity was calculated based on the fluorescence levels of the isotype control used. BGN is detected on the surface of these cells.

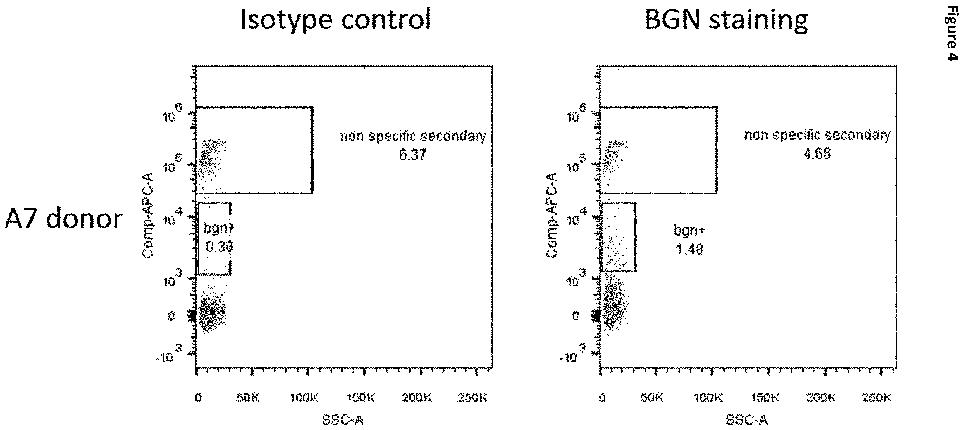

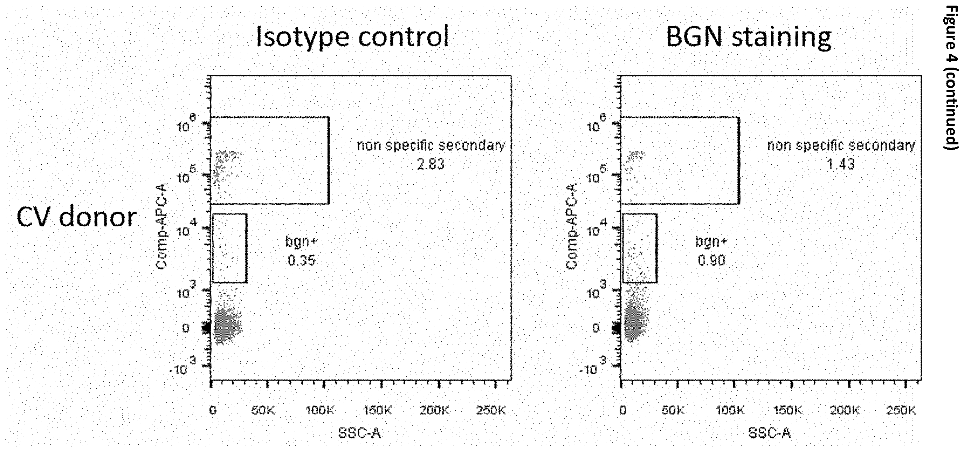

Fig. 4: BGN surface staining in the PBMCs acquired from the blood of two healthy donors (A7, CV). Positivity was calculated based on fluorescence levels of the isotype control used.

Fig. 5: BGN-TLR4 mediated activation of the inflammasome on the MDS-L cell line as detected by caspase 1 activity. MDS-L subpopulations were sorted and directly incubated overnight with 1µg/mL soluble BGN. Caspase 1 activity was measured for the 3 subpopulations of interest (SC1, SC2, TLR4) and was found statistically higher only for the TLR4 subpopulation after BGN stimulation. Additionally, the TLR4 subpopulation was cultured in the presence of possible inhibitors of this pathway (TGF-b1, TIRAP inhibitor, IFN-γ, IFN-α) with TGFb1 and IFN-γ showing the strongest inhibition. Moreover, TLR4 cells were also cultured with the addition of supernatant from the BGN expressing SC2 subpopulation, or directly cocultured with SC2 cells resulting in high caspase 1 activity without the addition of recombinant human BGN.

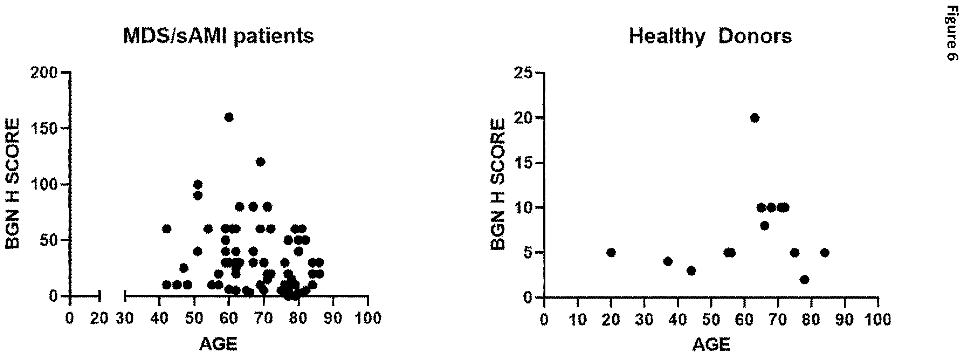

Fig. 6: Correlation of BGN h score (taking into account BGN positive cells and staining intensity) and age within the bone marrow of MDS/sAML patients and healthy donors as determined by immunohistochemistry.

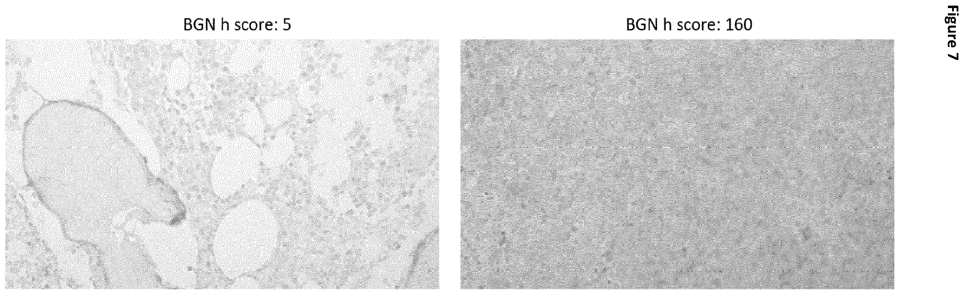

Fig. 7: Immunohistochemistry staining of BGN of the bone marrow from two MDS patients demonstrating the range of intensity. On the left, the biopsy of an MDS patient within healthy BGN expression range can be observed, in comparison to the highly expressing patient on the right.

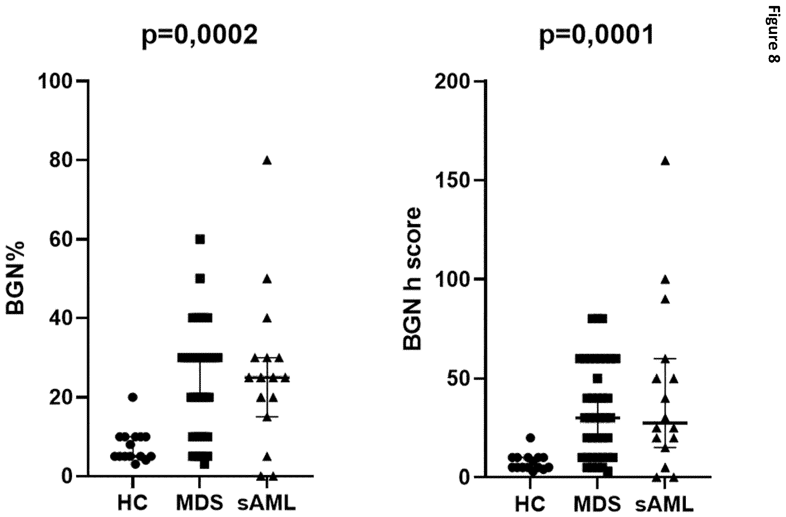

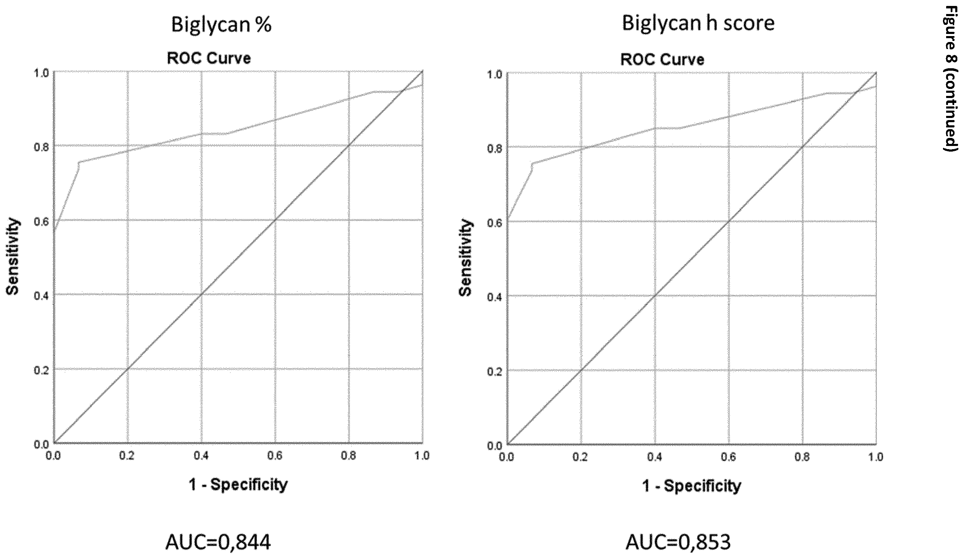

Fig. 8: Dot plots and ROC curves demonstrating the diagnostic value of BGN expression for MDS/sAML, taking into account only percentage of BGN positive cells (BGN%), or the BGN h score (cell positivity and intensity of staining). For both BGN% and BGN hscore the cutoff with maximum sensitivity and specificity is at 12,5 (Sens=0,69 Spec=1), with maximum values for healthy donors at hscoreₘₐₓ=20.

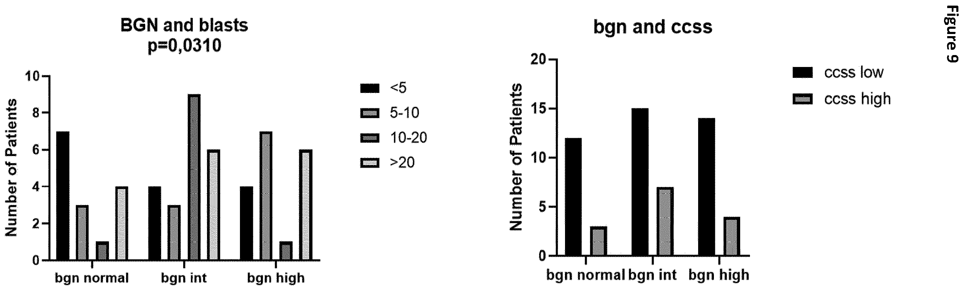

Fig. 9: Distribution of the BGN expression compared to the number of blasts within the bone marrow and the cytogenetic score (CCSS) of the patients. Patients' BGN expression is separated into normal (within healthy control maximum <20), intermediate (<=40) and high (>40).

## Claims

1. A method for identifying the need of a subject suffering from or being at risk of developing cancer to receive a cancer therapy, particularly a cancer immunotherapy, comprising determining the level of a small leucine-rich proteoglycan (SLRP), preferably biglycan (BGN), or a fragment thereof in a sample of the subject to be identified, wherein said level of SLRP, preferably BGN, in the sample of the subject to be identified is indicative of a need of said subject to receive a cancer therapy, particularly a cancer immunotherapy.

2. A method for the diagnosis, prognosis, risk assessment, risk stratification, therapy control and/or post-operative control of cancer in a subject comprising determining the level of a small leucine-rich proteoglycan (SLRP), preferably biglycan (BGN), or a fragment thereof in a sample of the subject to be identified, wherein said level of SLRP, preferably BGN, or a fragment thereof is indicative of said subject to suffer from or being at risk of developing cancer and/or a need of said subject to receive a cancer therapy, particularly a cancer immunotherapy.

3. The method of claim 1 or 2, wherein the method further comprises comparing the determined level of a SLRP, preferably BGN, or a fragment thereof to a reference level, preferably the reference level comprises the level of SLRP, preferably BGN, or a fragment thereof in a sample of at least one healthy subject, preferably the mean level of the SLRP, preferably BGN, or a fragment thereof in samples obtained from a healthy population.

4. The method of claim 3, wherein an increased level of SLRP, preferably BGN, or a fragment thereof in the sample from the subject to be identified compared to the reference level is indicative of a need of said subject to receive a cancer therapy, particularly a cancer immunotherapy.

5. The method of any of the preceding claims, wherein SLRP, preferably BGN, or a fragment thereof is expressed on the cell surface, preferably on the cell surface of hematopoietic cells.

6. The method of any of the preceding claims, wherein SLRP is selected from the group consisting of BGN and decorin (DCN), more preferably SLRP is BGN.

7. The method of any of the preceding claims, wherein the sample is selected from the group consisting of a blood sample, preferably a peripheral blood sample and serum, and a sample from bone marrow.

8. The method of any of the preceding claims, wherein the cancer is a hematopoietic cancer selected from one or more of myelodysplastic syndrome (MDS), acute myeloid leukemia (AML), particularly secondary acute myeloid leukemia (sAML), acute lymphoid leukemia (ALL), chronic lymphocytic leukemia (CLL), acute leukemia, B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, non-Hodgkin's lymphoma, Hodgkin's lymphoma, plasmablastic lymphoma, plasmacytoid dendritic cell neoplasm, and Waldenstrom macroglobulinemia.

9. The method of any of the preceding claims, wherein the cancer is selected from the group consisting of myelodysplastic syndrome (MDS) and acute myeloid leukemia (AML), particularly secondary acute myeloid leukemia (sAML) chronic myeloid leukemia (CML), acute lymphocytic leukemia (ALL) and chronic lymphocytic leukemia (CLL).

10. The method of any of the preceding claims, wherein the cancer is MDS or AML, particularly sAML.

11. The method of any of the preceding claims, wherein the cancer therapy is a cancer immunotherapy selected from the group consisting of chimeric antigen receptor (CAR) T-Cell therapy, chemotherapy and stem cell transplantation.
